# EUROPEAN PATENT APPLICATION

(11) **EP 1 860 891 A1**
(43) Date of publication of application: **28.11.2007**
(21) Application number: 06714318.0
(22) Date of filing: 22.02.2006
(51) Int. Cl.: H04Q 1/00

(54) **MEDICAL TOOL DELIVERY SYSTEM AND MEDICAL TOOL DELIVERY METHOD**

(30) Priority: 17.03.2005 JP 2005077768
(71) Applicant: Olympus Corporation, Tokyo 151-0072 (JP)
(72) Inventor: MORIYAMA, Hiroki, Olympus Medical Systems Corp., Tokyo 151-0072 (JP)
(74) Representative: von Hellfeld, Axel
(86) International application number: PCT/JP2006/303178
(87) International publication number: WO 2006/098125

(57) **Abstract**

A medical instrument distributing system of the present invention includes hospital terminals provided in multiple hospitals, a server placed in a manufacturer, which includes a computer system that processes orders/shipment of a disposable medical instrument based on inputted information that orders the disposable medical instrument from the hospital terminals. The hospital terminals and the server are connected by a wide area network to establish a system. In accordance with order information, ordered multiple disposable medical instruments are stored in a sterilization bag packages sealed after sterilization and are shipped to the ordering hospital. Thus, the number of complicated procedures in the hospital facilities can be reduced, and the distribution of medical instruments can be more improved.

## Description

### Technical Field

The present invention relates to a medical instrument distributing system and medical instrument distributing method for ordering and shipping a disposable medical instrument.

### Background Art

In recent years, various medical instruments have been developed and sold with the advance of medical technologies. These medical instruments may be reusable or disposable after an examination/treatment or a technique is performed. A reusable medical instrument undergoes cleaning and sterilization processing by an auto-clave, for example.

The reusable medical instruments include an endoscope, and the reusable endoscope may be used in combination with a disposable medical instrument such as an externally provided channel (which will be called "external channel" hereinafter), which is additionally adhered to an insertion section of the endoscope, which is disclosed in Japanese Unexamined Patent Application Publication No. 2002-330298, for example. Various kinds of disposable medical instruments are available such as treatment instruments and external channels, and the number and type of disposable instruments to be used may depend on the case to be treated and the endoscope to be used for the case. The frequency of use (general versatility) also depends on the type of medical instruments including ones to be used widely in accordance with the types of medical instruments and ones to be used for special cases only.

The applicable treatment instruments to be used for a case to be treated may have different lengths and/or outside diameters depending on the types of endoscopes (such as ones for the upper digestive tract, the lower digestive tract and the duodenum). The external channels as described above may also have different applicable channel inside diameters and lengths depending on the case to be treated and the type of endoscope to be used. Furthermore, since the outside diameter and length of a usable treatment instrument may depend on an external channel to be used if any, and various combinations thereof are possible.

In this way, various combinations may be possible between/among disposable medical instruments to be used in accordance with the cases to be treated and the types of endoscope to be used for the cases. Therefore, each of disposable medical instruments as described above is distributed in a separate sterilization bag package though repetitively usable medical instruments such as endoscopes are generally continuously placed in a hospital.

However, each of disposable instruments is separately stored to be distributed, and the frequency of use differs. Therefore, the medical facility side such as a hospital must perform stock control and order processing with particular attention to medical instruments or the combinations required for cases so as to keep them in stock or not to over-stock them, which disadvantageously results in complicated procedures and imposes a large burden on the medical facility side.

The present invention was made in view of the above issues, and it is an object of the present invention to provide a medical instrument distributing system and medical instrument distributing method that can reduce the number of complicated procedures in a medical facility and improve the distribution of medical instruments.

### Disclosure of Invention

### Means for Solving the Problem

A medical instrument distributing system as in Claim 1 of the present invention includes:
an instrument information extracting section that extracts, as a candidate for selection, information on a disposable medical instrument usable for a medical practice from multiple kinds of repetitively usable medical instruments based on information on the medical practice to be performed in the medical facility, which is obtained through a communication line;
an instrument shipment instructing section that, based on the selection information on the medical instrument selected from the candidates for selection of medical instruments and information related to the medical facility from which the selection information is obtained, performs shipment instruction to store and ship a medical instrument in accordance with the selected medical instrument in a single sterilization bag package to the medical facility; and
a communicating section that transmits and receives information with a terminal provided in a medical facility.

A medical instrument distributing method as in Claim 8 of the present invention includes the steps of:
obtaining information on a medical practice to be performed in a medical facility through a communication line;
extracting, as a candidate for selection, information on a medical instrument usable for the medical practice from multiple kinds of medical instruments based on the obtained information on the medical practice;
providing the information on the medical instrument as the candidate for selection to the medical facility;
obtaining, from the medical facility, selection information on the medical instrument selected from candidates for selection of medical instruments, which are provided to the medical facility; and
instructing to store and ship the medical instrument in accordance with the selected medical instrument in a single sterilization bag package and ship the package to the medical facility based on the medical instrument selection information and information on the medical facility from which the selection information is obtained.

### Brief Description of the Drawings

Fig. 1 is a configuration diagram showing the configuration of a disposable medical instrument distributing system according to Embodiment 1 of the present invention;
Fig. 2 is a diagram showing a sterilization sealed pack to be shipped from the product shipment section in Fig. 1;
Fig. 3 is a flowchart showing an operation of the disposable medical instrument distributing system in Fig. 1;
Fig. 4 is a diagram showing a product ordering window developed on a hospital terminal in the processing in Fig. 3;
Fig. 5 is a diagram showing a field-of-technique selection window developed on a hospital terminal in the processing in Fig. 3;
Fig. 6 is a diagram showing a type-of-examination selection window developed on a hospital terminal in the processing in Fig. 3;
Fig. 7 is a diagram showing an endoscope list window developed on a hospital terminal in the processing in Fig. 3;
Fig. 8 is a diagram showing a product selection window developed on a hospital terminal in the processing in Fig. 3;
Fig. 9 is a diagram showing a first hierarchical product group list window developed on a hospital terminal in the processing in Fig. 3;
Fig. 10 is a diagram showing a second hierarchical product group list window developed on a hospital terminal in the processing in Fig. 3;
Fig. 11 is a diagram showing a product group list window developed on a hospital terminal in the processing in Fig. 3; and
Fig. 12 is a diagram showing an order confirmation window developed on a hospital terminal in the processing in Fig. 3.

### Best Mode for Carrying Out the Invention

Embodiments of the present invention will be described below with reference to the drawings.

### EMBODIMENT 1

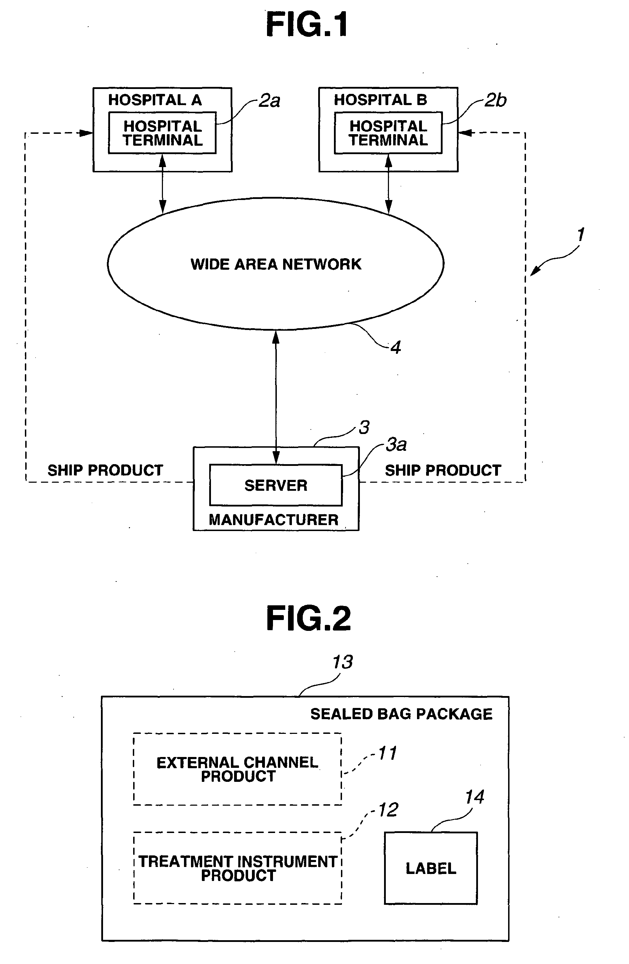

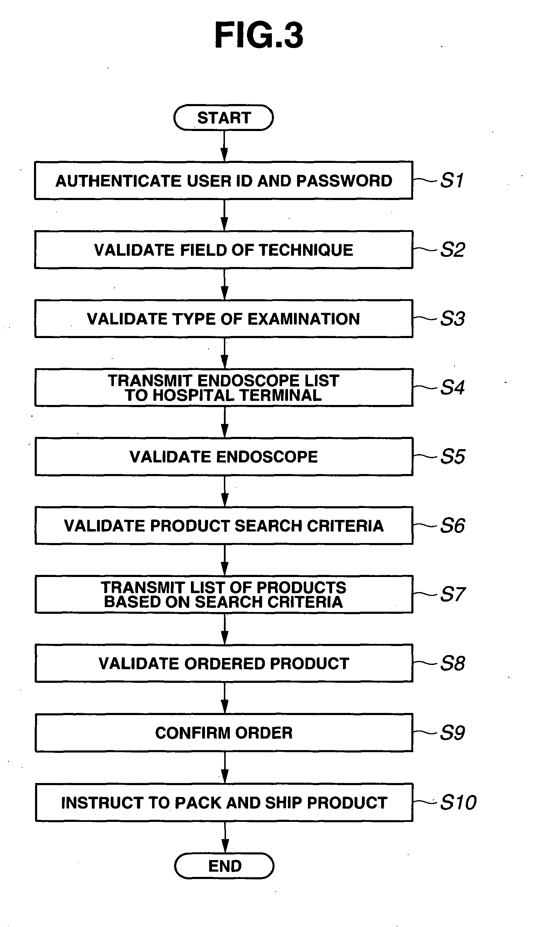

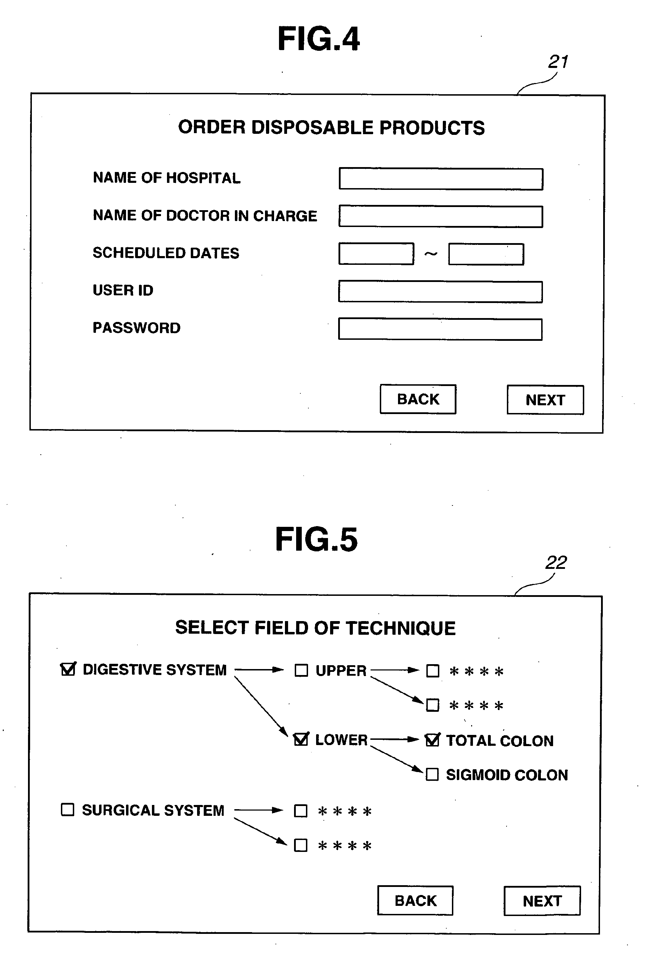

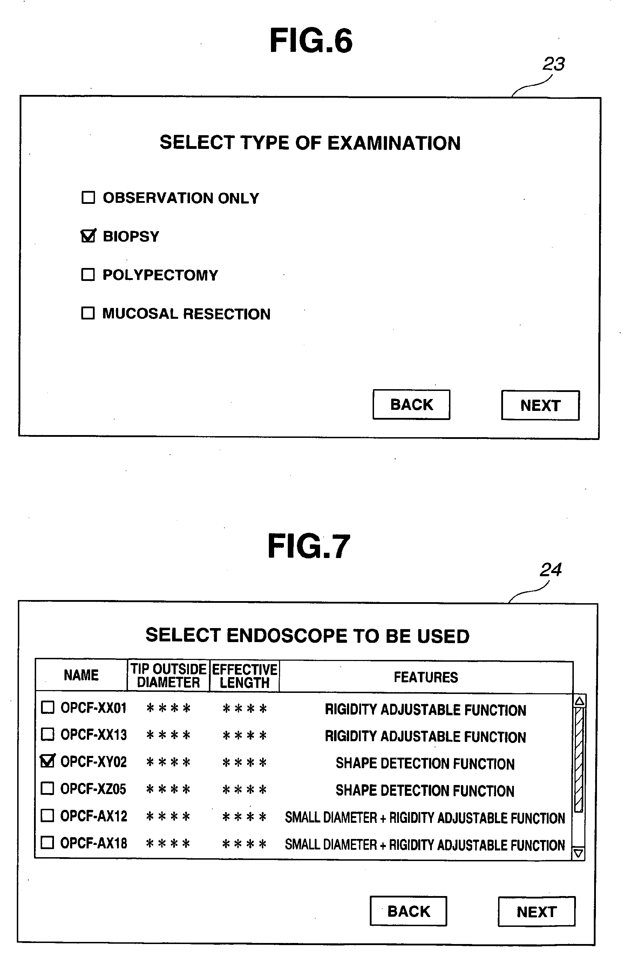

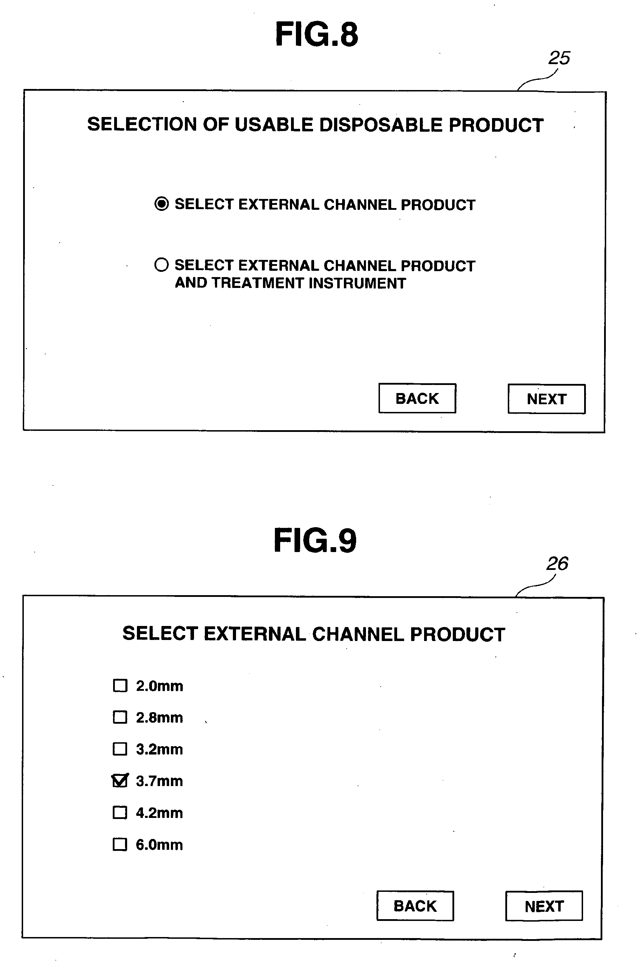

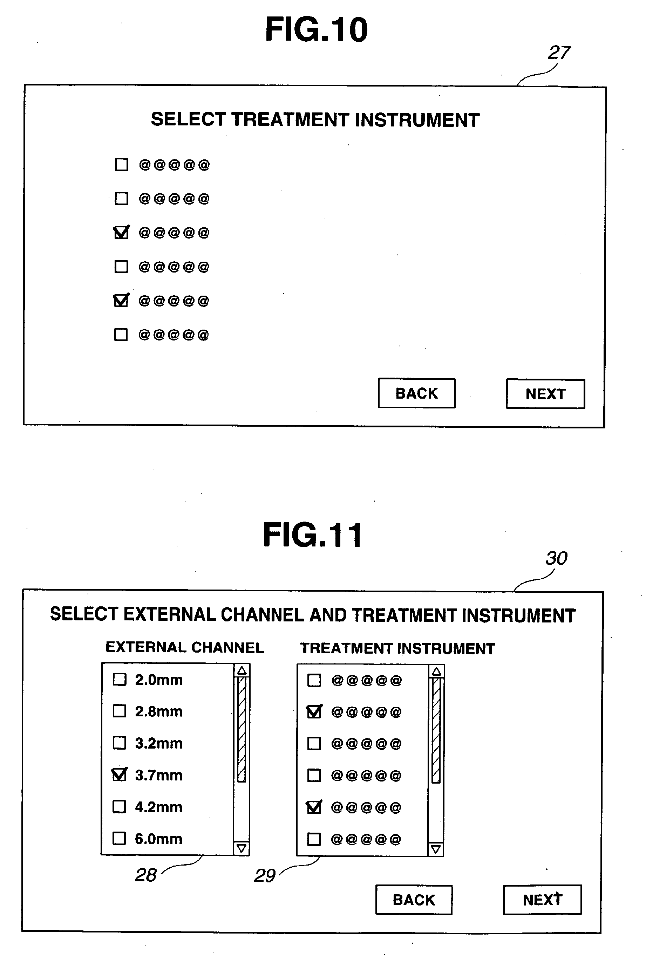

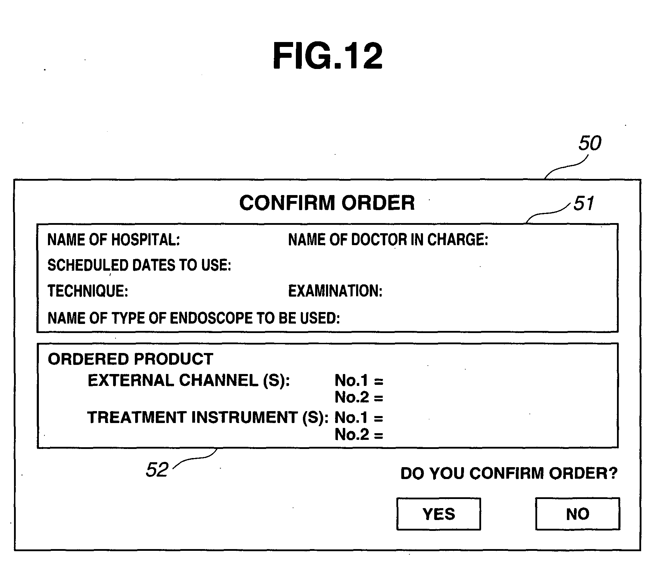

Figs. 1 to 12 relate to Embodiment 1 of the present invention. Fig. 1 is a configuration diagram showing the configuration of a disposable medical instrument distributing system, Fig. 2 is a diagram showing a sterilization sealed pack to be shipped from the product shipment section in Fig. 1, Fig. 3 is a flowchart showing an operation of the disposable medical instrument distributing system in Fig. 1, Fig. 4 is a diagram showing an product ordering window developed on a hospital terminal in the processing in Fig. 3, Fig. 5 is a diagram showing a field-of-technique selection window developed on a hospital terminal in the processing in Fig. 3, Fig. 6 is a diagram showing a type-of-examination selection window developed on a hospital terminal in the processing in Fig. 3, Fig. 7 is a diagram showing a endoscope list window developed on a hospital terminal in the processing in Fig. 3, Fig. 8 is a diagram showing a product selection window developed on a hospital terminal in the processing in Fig. 3, Fig. 9 is a diagram showing a first hierarchical product group list window developed on a hospital terminal in the processing in Fig. 3, Fig. 10 is a diagram showing a second hierarchical product group list window developed on a hospital terminal in the processing in Fig. 3, Fig. 11 is a diagram showing a product group list window developed on a hospital terminal in the processing in Fig. 3, and Fig. 12 is a diagram showing a order confirmation window developed on a hospital terminal in the processing in Fig. 3.

As shown in Fig. 1, a medical instrument distributing system 1 of the present embodiment is placed in multiple medical facilities (such as hospitals), which are clients or ordering parties. The medical instrument distributing system 1 includes hospital terminals 2a, 2b and so on, and a server 3a. The hospital terminals 2a, 2b and so on function as client terminals for inputting an order for a disposable medical instrument in accordance with a case subject to a technique. The server 3a is placed in a party that receives the order such as a manufacturer 3 that manufactures a medical instrument (which will be called manufacturer 3 hereinafter) for performing order and shipment processing in response to information on an order for a medical instrument from the hospital terminals 2a, 2b and so on. The hospital terminals 2a, 2b and so on and server 3 are connected over a wide area network (which may be the Internet, but may be any communication line).

The server 3a placed in the manufacturer 3 has a communication apparatus function as a communication section and receives an order for a medical instrument from the hospital terminals 2a, 2b and so on over the wide area network 4 by the communication function. The server 3a has functions of performing order processing for a medical instrument in accordance with the type of an endoscope owned by the hospital and the case subject to a technique and performing processing for shipping the ordered medical instrument to the hospital.

In other words, the server 3a performs processing for storing multiple disposable medical instruments (including an external channel instrument 11 and a treatment instrument 12 to be used along with the external channel instrument 11, for example) to be used in accordance with the case related to the order into one sterilization bag package, as shown in Fig. 2. A label 14 having information on the client and information on the stored medical instrument is attached to the outer surface of the sterilization bag package 13. The server 3a further performs processing of shipping the sterilization bag package 13 with the label 14 attached to the ordering medical facility such as a hospital.

Here, the label 14 is not typically necessarily attached to the outer surface of the bag package but may be placed within the sterilization bag package if visible or, alternatively, may be written on the sterilization bag package 13 directly by processing like printing.

An operation of the medical instrument distributing system 1 of the present embodiment having the configuration will be described.

In response to an access for ordering a disposable medical instrument from the hospital terminal 2a, 2b or the like to the server 3a over the wide area network 4, user authentication processing based on a user ID and a password is performed on the hospital terminal 2a, 2b or the like in step S1, as shown in Fig. 3.

More specifically, the server 3a transmits information (or an image) of a disposable product ordering window 21 having an authentication frame, as shown in Fig. 4, to the hospital terminal 2a, 2b or the like. Then, the server 3a authenticates the user by receiving multiple entered items (such as the name of the hospital, name of the doctor in charge (name of the ordering person), scheduled date, user ID and password) on the disposable product ordering window 21.

Fig. 4 shows the configuration in which the items such as the name of the hospital, name of the doctor in charge (name of the ordering person) and scheduled date are entered simultaneously with the user ID and password, obviously these items may be entered after the user authentication.

Then, in step S2 after the user authentication completes, the server 3a transmits a field-of-technique selection window 22 for selecting the field of a planned technique as shown in Fig. 5 to the hospital terminal 2a, 2b or the like and receives and validates the information on the selection of the field of the technique on the field-of-technique selection window 22.

In step S3, in response to the receipt of the selection information, the server 3 a transmits a type-of-examination selection window 23 for selecting the type of the planned examination as shown in Fig. 6 to the hospital terminal 2a, 2b or the like and receives and validates the selection information on the type of the examination of the type-of-examination selection window 23.

After the user ID, selection information on the field of the technique, and selection information on the type of the examination are obtained in this way, the server 3a next in step S4 retrieves an endoscope applicable to the selected technique and examination by an apparatus information extracting function as an apparatus information extracting section and transmits an applicable endoscope list window 24 as shown in Fig. 7 to the hospital terminal 2a, 2b or the like.

In the present embodiment case, product information of endoscopes available on the market are pre-stored in a database in a storage device (not shown) in the server 3a. Then, the hospital terminal 2a, 2b or the like displays a list of the names of the endoscopes, and the ordering party such as a hospital is configured to select an endoscope to be used for a case from the list.

Next, the server 3a in step S5 receives and validates the information on the selection of the endoscope planned to be used, which is inputted from the hospital terminal 2a, 2b or the like by the endoscope list window 24.

In response to the receipt of the inputted endoscope selection information, the server 3a in step S6 transmits a product selection window 25 for selecting the method for selecting a disposable product applicable to the endoscope planned to be used, as shown in Fig. 8 to the hospital terminal 2a, 2b or the like and receives and validates the product selection criteria information entered by the product selection window 25.

Fig. 8 shows the example in which hierarchical selection information for first selecting the external channel applicable to the endoscope to be planned to use and selecting a treatment instrument usable with the selected external channel (that is, the selection items of external channel products) and same level selection information for simultaneously selecting an external channel and treatment instrument applicable to the endoscope planned to be used (that is, the selection items of external channel products and treatment instruments) are prepared.

Steps S1 to S6 above are steps for obtaining order information. The steps are performed by an order information obtaining function that the sever 3a has.

Then, the product order receiving section 3a in step S7 retrieves usable disposable products in accordance with the product selection criteria information and transmits a list window showing a product group having a list of products to the hospital terminal 2a, 2b or the like.

More specifically, in Fig. 8, for example, when the hierarchical selection information (that is, selection items of external channel products) is selected as the product selection criteria information, the server 3a transmits a first hierarchical product group list window 26 having a product group list of usable external channel products as shown in Fig. 9 to the hospital terminal 2a, 2b or the like. Then, when an external channel product is selected by an ordering party, the server 3a transmits a second hierarchical product group list window 27 having a product group list of treatment instruments applicable to the external channel product as shown in Fig. 10 to the hospital terminal 2a, 2b or the like.

In Fig. 8, when the same-level selection information (that is, selection items of external channel products and treatment instruments) for simultaneously selecting an external channel and treatment instrument applicable to the endoscope planned to use is selected, the server 3a transmits a product group list window 30 having a product group list 28 of external channel products and a product group list 29 of treatment instruments usable for the endoscope, as shown in Fig. 11, to the hospital terminal 2a, 2b or the like.

Step S7 above is a step for extracting and developing an instrument list and is performed by an instrument list extracting/developing function as an instrument information extracting section that the server 3a has.

Then, the server 3 a receives and validates the product information of ordered products (that is, an external channel product and treatment instrument) desired by a user to order on the product group list window in step S8 from the hospital terminal 2a, 2b or the like.

After the product information is validated in this way, the server 3a in step S9 transmits an order confirmation window 50 as shown in Fig. 12 to the hospital terminal 2a, 2b or the like. The order confirmation window 50 displays user information 51 and ordered product information 52 entered by the user, and the confirmation on the user information 51 and ordered product information 52 is received and validated.

Steps S8 and S9 above are order information validating steps and are performed by an order information validating function that the server 3 a has.

Then, the after the confirmation on the user information 51 and ordered product information 52 by the client is validated, the server 3a implements an instrument shipment instructing function as an instrument shipment instructing section that the server 3a has. In other words, in step S10, which is the instrument shipment instructing step, a product packing instruction and shipment instruction including the user information 51 and ordered product information 52 are transmitted from the server 3a to a product delivery department (not shown). The product delivery department packs the products and arranges the shipment based on the transmitted instruction.

In response to th shipment instruction, the product shipment department stores ordered products in one sterilization bag package 13 (refer to Fig. 2). The label 14 having the user information 51 and ordered product information 52 as client information is attached to the sterilization bag package 13.

Then, the product shipment department ships the sterilization bag package 13 storing the ordered products to the client hospital.

In the present embodiment, a user hospital can use the hospital terminal 2a, 2b or the like to access the server 3 a easily over the wide area network 4. Furthermore, since the server 3a provides a user with a list of disposable products in accordance with the usage such as the planned case based on the hierarchical selection method or same-level selection method, the user can easily combine and select optimum products.

In other words, a medical facility such as a hospital, which is a user, can greatly reduce the labor for the stock control, and the manufacturer side can simplify order processing on a combination of disposable products.

Furthermore, since a product delivery department delivers the selected combination of products stored in one sterilization bag package 13 with the label 14 attached having the user information 51 and ordered product information 52 to an ordering hospital, the management on the user side can be performed in sterilization sealed packs. The label 14 also makes the content of the sterilization sealed pack easily visible.

Having described the present embodiment by using external channel products and treatment instruments as disposable medical instruments, for example, the invention is not limited to the combination. Multiple treatment instruments to be used for one case may be combined.

As described in the present embodiment, the present invention can reduce the number of complicated procedures in a medical facility and allows easy ordering of disposable medical instruments, which are applicable to reusable medical apparatus.

The present invention is not limited to the embodiments above, and various changes, alteration and so on can be made thereto without departing from the scope and spirit of the present invention.

## Claims

1. A medical instrument distributing system comprising:
an instrument information extracting section that extracts, as a candidate for selection, information on a disposable medical instrument usable for a medical practice from multiple kinds of repetitively usable medical instruments based on information on the medical practice to be performed in the medical facility, which is obtained through a communication line;
an instrument shipment instructing section that, based on the selection information on the medical instrument selected from the candidates for selection of medical instruments and information related to the medical facility from which the selection information is obtained, performs shipment instruction to store and ship a medical instrument in accordance with the selected medical instrument in a single sterilization bag package to the medical facility; and
a communicating section that transmits and receives information with a terminal provided in a medical facility.

2. The medical instrument distributing system according to Claim 1, further comprising an apparatus information extracting section that extracts information on a medical apparatus usable for the medical practice, wherein:
the apparatus information extracting section extracts, as a candidate for selection, information on a medical apparatus usable for a medical practice based on the information on the medical practice to be performed in the medical facility.

3. The medical instrument distributing system according to Claim 2, wherein:
the medical instrument is a medical instrument usable in combination with the medical apparatus; and
the instrument information extracting section extracts, as a candidate for selection, information on the medical instrument usable in combination with a medical apparatus selected from the information on the medical apparatus provided to the medical facility.

4. The medical instrument distributing system according to Claim 2 or 3, wherein:
the medical apparatus is an endoscope; and
the apparatus information extracting section extracts, as a candidate for selection, information on the endoscope usable for a medical practice in the medical facility based on the information, which relates to the medical practice using the endoscope and is obtained from the medical facility.

5. The medical instrument distributing system according to Claim 4, further comprising a storage device that stores the information relating to a medical practice, the information on a medical instrument and the information on a medical apparatus.

6. The medical instrument distributing system according to any one of Claims 1 to 5, wherein the medical instrument extracting section has:
a first instrument list developing section that develops a first list of medical instruments; and
a second instrument list developing section that hierarchically develops, for the first medical instruments, a second list of medical instruments usable in combination with the first medical instruments.

7. The medical instrument distributing system according to any one of Claims 1 to 6, further comprising:
a label creating section that creates a label having information on a medical instrument to be stored in the sterilization bag package.

8. A medical instrument distributing method comprising the steps of:
obtaining information on a medical practice to be performed in a medical facility through a communication line;
extracting, as a candidate for selection, information on a medical instrument usable for the medical practice from multiple kinds of medical instruments based on the obtained information on the medical practice;
providing the information on the medical instrument as the candidate for selection to the medical facility;
obtaining, from the medical facility, selection information on the medical instrument selected from candidates for selection of medical instruments, which are provided to the medical facility; and
instructing to store and ship the medical instrument in accordance with the selected medical instrument in a single sterilization bag package and ship the package to the medical facility based on the medical instrument selection information and information on the medical facility from which the selection information is obtained.

9. The medical instrument distributing method according to Claim 8, wherein the step of extracting information on a medical instrument has:
a first instrument list developing step of developing a first list of medical instruments; and
a second instrument list developing step of hierarchically developing, to the first medical instruments, a second list of medical instruments usable in combination with the first medical instruments.
